# EUROPEAN PATENT SPECIFICATION

(11) **EP 1 787 644 B1**
(45) Date of publication and mention of the grant of the patent: **18.08.2010**
(21) Application number: 06123574.3
(22) Date of filing: 07.11.2006
(51) Int. Cl.: A61K 9/00, A61K 31/19, A61K 31/4164

(54) **Otological solution and method for its preparation**
Otologische Lösung und Verfahren zu ihrer Herstellung
Solution otologique et procédé de préparation de celle-ci

(30) Priority: 10.11.2005 IT RM20050561
(43) Date of publication of application: 23.05.2007
(73) Proprietor: D.M.G. Italia Srl, 00040 Pomezia (RM) (IT)
(72) Inventor: Mercuri, Luigi, I-00040, Pomezia (RM) (IT)
(74) Representative: Sarpi, Maurizio

(56) References cited:
- WO-A2-99/20261
- US-A- 5 461 068
- US-A- 5 900 393
- US-A1- 2005 239 722
- HIRSCH B E: "INFECTIONS OF THE EXTERNAL EAR" 1 May 1992 (1992-05-01), AMERICAN JOURNAL OF OTOLARYNGOLOGY, W.B. SAUNDERS, PHILADELPHIA, PA, US, PAGE(S) 145-155 , XP000607313 ISSN: 0196-0709 * page 147, column 1, paragraph 4 - column 2, paragraph 1 *
- FAIRBANKS: "Otic Topical Agents" OTOLARYNGOLOGY: HEAD AND NECK SURGERY, vol. 88, 1980, pages 327-331, XP009078974
- F. C. ODDS: "Laboratory evaluation of antifungal agents: a comparative study of five imidazole derivatives of clinical importance" JOURNAL OF ANTIMICROBIAL CHEMOTHERAPY, vol. 6, 1980, pages 749-761, XP009079281

## Description

The present invention relates to the pharmaceutical sector and in particular to a composition in the form of a solution, with antimycotic, antisporigenic, and lenitive action usable in the otological field, as well as to the method for its preparation.

As is known, there are numerous irritative forms of the auditive apparatus caused by mycoses and/or allergic causes, such as, for example, allergy to chlorine. In particular, said pathological conditions develop above all in the summer period and with greater frequency in subjects who practice water sports.

Currently, different types of antimycotics are commercially available, but none of these is suitable for use in the otological field in so far as they do not present the necessary transparency and limpidity, which is fundamental for enabling clinical examinations of the auditive duct.

For instance, it is known from US Patent 5.461.068 a stable solution for a topical treatment of fungal infections which comprises a solvent system based on acetic acid and a therapeutically amount of miconazole nitrate. However said antifungal solution is suitable for use only dermatological. Acetic acid is known to treat infections of the ear (Barry et al., Am. J. otolaryngology 1992, 13 (3), 145-155).

In addition, no antimycotics are known that have an antisporigenic action such as to prevent recidivation due to the survival of the spores in the external auditive duct.

In the light of what is known to the state of the art and with due consideration of the problems set forth above, there is particularly felt the need to provide a preparation with antimycotic, antisporigenic, and lenitive activity specifically designed for otological use and for this use completely transparent and limpid.

In particular, considering the difficulty linked to obtaining a limpid and transparent solution on account of the low solubility in water of known antimycotic components and of their poor activity in regard to spores, a preparation is provided in the form of a limpid and transparent solution, comprising in combination:
1) climbazole with antibacterial and antimycotic activity, particularly active in regard to *candida albicans* and *aspergillus niger;* and
2) acetic acid having activity against sporigenic forms,
   in a concentration determined experimentally in order to obtain an effect against spores in the times of application of the product in the external auditive canal, without any side effects.

Optionally, added to the aforesaid composition is a mixture of essential oils *(origanum vulgare, cinnamonum zeylanicum, rosmarinus officinalis, lavandula officinalis, menta piperita, citrus limonum, hydrastis canadensis, olea europea),* having an antimycotic action synergistic with climbazole for reinforcing the antimycotic activity.

Consequently, forming a subject of the present invention is a composition, in the form of a limpid and transparent solution, comprising climbazole in a concentration of between 0.2 wt% and 5 wt% and acetic acid in a concentration of between 0.5% and 20 wt%, to which is optionally added a mixture of essential oils *(origanum vulgare, cinnamonum zeylanicum, rosmarinus officinalis, lavandula officinalis, menta piperita, citrus limonum, hydrastis canadensis, olea europea),* in a concentration of between the 0.05% and 5 wt%.

Also added are the following components:
a) propylene glycol as lubricating vehicle having characteristics of solvent;
b) glycerine with softening and hydrating action;
c) dipotassium glycyrrhizinate with antiinflammatory and lenitive activity; and
d) allantoine with hydrating and lenitive activity.

In a preferred embodiment, the solution forming the subject of the present invention contains, for every 100 grams:

| | |
|---|---|
| PROPYLENE GLYCOL | 60 g |
| GLYCERINE | 5.0 g |
| CLIMBAZOL | 1 g |
| DEMINERALIZED WATER | 29.4 g |
| DIPOTASSIUM GLYCYRRHIZINATE | 0.2 g |
| ALLANTOINE | 0.1 g |
| MIXTURE OF ESSENTIAL OILS | 0.3 g |
| ACETIC ACID, OFFICIAL PHARMACOPOEIA | 4 g |

The composition forming the subject of the present invention was subjected to tests of stability by accelerated ageing at a temperature around 37°C, and maintenance of the chemico-physical parameters was found.

Forming a further subject of the invention is the method for preparation of the composition so far described, comprising the following steps:
A) mixing propylene glycol with glycerine;
B) adding to the mixture obtained in step A) climbazol under stirring, heating to around 50°C to accelerate solubilization;
C) cooling the mixture obtained in step B) until a perfectly limpid and transparent preparation is obtained;
D) when the preparation obtained in step C) is perfectly limpid, adding water slowly and under stirring in order to maintain the limpidity of the preparation;
E) adding to the preparation obtained in step D) 18-beta glycyrrhetinic acid, dipotassium salt, and allantoine, stirring up to complete solubilization;
F) adding to the preparation obtained in step E) the acetic acid under stirring; and
G) adjusting the pH of the composition obtained in step F) up to the value 4.
   The method optionally envisages an additional step
H) following step C) and prior to step D) in which the mixture of essential oils is added to the solution obtained in step C) and solubilized therewith.

## Claims

1. An antimycotic and antisporigenic composition, in the form of a limpid and transparent solution, comprising climbazole and acetic acid, for use in the treatment of irritative forms of the auditive apparatus caused by mycoses and/or allergic causes.

2. The antimycotic and antisporigenic composition, in the form of a limpid and transparent solution for use, according to Claim 1, **characterized in that** climbazole is in a concentration of between 0.2 wt% and 5 wt%.

3. The antimycotic and antisporigenic composition, in the form of a limpid and transparent solution for use, according to Claim 1, **characterized in that** the acetic acid is in a concentration of between 0.5% and 20 wt%.

4. The antimycotic and antisporigenic composition, in the form of a limpid and transparent solution for use, according to Claims 1 to 3, **characterized in that** added thereto is a mixture of essential oils.

5. The antimycotic and antisporigenic composition, in the form of a limpid and transparent solution for use, according to Claim 4, **characterized in that** the mixture of essential oils is in a concentration of between 0.05% and 5 wt%.

6. The antimycotic and antisporigenic composition, in the form of a limpid and transparent solution, according to Claim 4, **characterized in that** the mixture
of essential oils comprises *origanum vulgare, cinnamonum zeylanicum, rosmarinus officinalis, lavandula officinalis, menta piperita, citrus limonum, hydrastis canadensis, olea europa*.

7. The antimycotic and antisporigenic composition, in the form of a limpid and transparent solution for use, according to any one of the preceding claims, **characterized in that** added thereto is at least one of the components comprised in the group consisting of: propylene glycol, glycerine, dipotassium glycyrrhizinate, allantoine.

8. A limpid and transparent solution for use in the treatment of irritative forms of the auditive apparatus caused by mycoses and/or allergic causes having the following composition for every 100 grams of final product:
| | |
|---|---|
| PROPYLENE GLYCOL | 60 g |
| GLYCERINE | 5.0 g |
| CLIMBAZOLE | 1 g |
| DEMINERALIZED WATER | 29.4 g |
| DIPOTASSIUM GLYCYRRHIZINATE | 0.2 g |
| ALLANTOINE | 0.1 g |
| MIXTURE OF ESSENTIAL OILS | 0.3 g |
| ACETIC ACID, OFFICIAL PHARMACOPOEIA | 4 g |

9. A method for the preparation of the composition of any one of Claims 1 to 8, comprising the following steps:
A) mixing propylene glycol with glycerine;
B) adding to the mixture obtained in step A) climbazole under stirring, heating to around 50°C to accelerate solubilization;
C) cooling the mixture obtained in step B) until a perfectly limpid and transparent preparation is obtained;
D) when the preparation obtained in step C) is perfectly limpid, adding water slowly and under stirring in order to maintain the limpidity of the preparation;
E) adding to the preparation obtained in step D) 18-beta glycyrrhetinic acid, dipotassium salt, and allantoine, stirring up to complete solubilization;
F) adding the acetic acid under stirring to the preparation obtained in step E);
G) adjusting the pH of the composition obtained in step F) up to the value 4.

10. The method according to Claim 9, **characterized in that** it optionally envisages an additional step H) following step C) and prior to step D), in which the mixture of essential oils is added to the solution obtained in step C) and solubilized therewith.

## Patentansprüche

1. Antimykotische und gegen Sporen wirksame Zusammensetzung in Form einer klaren und durchsichtigen Lösung, enthaltend Climbazol und Essigsäure, zur Verwendung bei der Behandlung von Reizformen des auditiven Systems, welche durch Mykosen und/oder allergische Ursachen ausgelöst wurden.

2. Antimykotische und gegen Sporen wirksame Zusammensetzung in Form einer klaren und durchsichtigen Lösung zur Verwendung nach Anspruch 1, **dadurch gekennzeichnet, dass** Climbazol in einer Konzentration von 0,2 Gew.-% bis 5 Gew.-% vorliegt.

3. Antimykotische und gegen Sporen wirksame Zusammensetzung in Form einer klaren und durchsichtigen Lösung zur Verwendung nach Anspruch 1, **dadurch gekennzeichnet, dass** die Essigsäure in einer Konzentration von 0,5 Gew.-% bis 20 Gew.-% vorliegt.

4. Antimykotische und gegen Sporen wirksame Zusammensetzung in Form einer klaren und durchsichtigen Lösung zur Verwendung nach einem der Ansprüche 1 bis 3, **dadurch gekennzeichnet, dass** dieser eine Mischung ätherischer Öle zugesetzt ist.

5. Antimykotische und gegen Sporen wirksame Zusammensetzung in Form einer klaren und durchsichtigen Lösung zur Verwendung nach Anspruch 4, **dadurch gekennzeichnet, dass** die Mischung ätherischer Öle in einer Konzentration von 0,05 Gew.-% bis 5 Gew.-% vorliegt.

6. Antimykotische und gegen Sporen wirksame Zusammensetzung in Form einer klaren und durchsichtigen Lösung nach Anspruch 4, **dadurch gekennzeichnet, dass** die Mischung ätherischer Öle *Origanum vulgare, Cinnamonum zeylanicum, Rosmarinus officinalis, Lavandula officinalis, Menta piperita, Citrus limonum, Hydrastis canadensis, Olea europea* enthält.

7. Antimykotische und gegen Sporen wirksame Zusammensetzung in Form einer klaren und durchsichtigen Lösung zur Verwendung nach einem der vorangegangenen Ansprüche, **dadurch gekennzeichnet, dass** dieser wenigstens einer der Bestandteile zugesetzt ist, der in der Gruppe, bestehend aus: Propylenglykol, Glyzerin, Dikaliumglycyrrhizinat, Allantoin enthalten ist.

8. Klare und durchsichtige Lösung zur Verwendung zur Behandlung von Reizformen des auditiven Systems, welche durch Mykosen und/oder allergische Ursachen ausgelöst wurden, mit der folgenden Zusammensetzung pro 100 Gramm des Endprodukts:
| | |
|---|---|
| PROPYLENGLYKOL | 60 g |
| GLYZERIN | 5,0 g |
| CLIMBAZOL | 1 g |
| ENTMINERALISIERTES WASSER | 29,4 g |
| DIKALIUMGLYCYRRHIZINAT | 0,2 g |
| ALLANTOIN | 0,1 g |
| MISCHUNG ÄTHERISCHER ÖLE | 0,3 g |
| ESSIGSÄURE, OFFIZIELLES ARZNEIBUCH | 4 g |

9. Verfahren zum Herstellen der Zusammensetzung gemäß einem der Ansprüche 1 bis 8, umfassend die folgenden Schritte:
A) Mischen von Propylenglykol mit Glyzerin;
B) Zusetzen von Climbazol zu der in Schritt A) erhaltenen Mischung unter Rühren, Erwärmen auf etwa 50 °C, um eine Auflösung zu beschleunigen;
C) Kühlen der in Schritt B) erhaltenen Mischung, bis eine vollkommen klare und durchsichtige Zubereitung erhalten wird;
D) wenn die in Schritt C) erhaltene Zubereitung vollkommen klar ist, langsames Zusetzen von Wasser unter Rühren, um die Klarheit der Zubereitung zu erhalten;
E) Zusetzen von 1 8-beta-Glycyrrhetinsäure, Dikaliumsalz und Allantoin zu der in Schritt D) erhaltenen Zubereitung und Rühren, um eine vollständige Auflösung zu erreichen;
F) Zusetzen der Essigsäure zu der in Schritt E) erhaltenen Zubereitung unter Rühren;
G) Einstellen des pH der in Schritt F) erhaltenen Zusammensetzung auf einen Wert von 4.

10. Verfahren nach Anspruch 9, **dadurch gekennzeichnet, dass** optional ein weiterer Schritt H) nach Schritt C) und vor Schritt D) vorgesehen ist, in welchem der in Schritt C) erhaltenen Lösung die Mischung ätherischer Öle zugesetzt und in dieser gelöst wird.

## Revendications

1. Composition antimycotique et antisporogénique, sous la forme d'une solution limpide et transparente, comprenant du climbazole et de l'acide acétique, pour une utilisation dans le traitement de formes irritatives de l'appareil auditif provoquées par des mycoses et/ou des causes allergiques.

2. Composition antimycotique et antisporogénique, sous la forme d'une solution limpide et transparente pour une utilisation selon la revendication 1, **caractérisée en ce que** le climbazole est à une concentration entre 0,2 % en poids et 5 % en poids.

3. Composition antimycotique et antisporogénique, sous la forme d'une solution limpide et transparente pour une utilisation selon la revendication 1, **caractérisée en ce que** l'acide acétique est à une concentration entre 0,5 % et 20 % en poids.

4. Composition antimycotique et antisporogénique, sous la forme d'une solution limpide et transparente pour une utilisation selon les revendications 1 à 3, **caractérisée en ce qu'**il lui est ajouté un mélange d'huiles essentielles.

5. Composition antimycotique et antisporogénique, sous la forme d'une solution limpide et transparente pour une utilisation selon la revendication 4, **caractérisée en ce que** le mélange d'huiles essentielles est à une concentration entre 0,05 %

6. Composition antimycotique et antisporogénique, sous la forme d'une solution limpide et transparente selon la revendication 4, **caractérisée en ce que** le mélange des huiles essentielles comprend *Origanum vulgare, Cinnamonum zeylanicum, Rosmarinus officinalis, Lavandula officinalis, Menta piperita, Citrus limonum, Hydrastis canadensis, Olea europea.*

7. Composition antimycotique et antisporogénique, sous la forme d'une solution limpide et transparente pour une utilisation selon l'une quelconque des revendications précédentes, **caractérisée en ce qu'**il lui est ajouté au moins l'un des composants compris dans le groupe constitué par : le propylène glycol, la glycérine, le glycyrrhizinate dipotassique, l'allantoïne.

8. Solution limpide et transparente pour une utilisation dans le traitement de formes irritatives de l'appareil auditif provoquées par des mycoses et/ou des causes allergiques, ayant la composition suivante pour 100 grammes de produit final :
| | |
|---|---|
| propylène glycol | 60 g |
| glycérine | 5,0 g |
| climbazole | 1 g |
| eau déminéralisée | 29,4 g |
| glycyrrhizinate dipotassique | 0,2 g |
| allantoïne | 0,1 g |
| mélange d'huiles essentielles | 0,3 g |
| acide acétique, pharmacopée officielle | 4 g |

9. Méthode de préparation de la composition selon l'une quelconque des revendications 1 à 8, comprenant les étapes suivantes :
A) le mélange du propylène glycol avec la glycérine ;
B) l'addition au mélange obtenu dans l'étape A) du climbazole sous agitation, chauffage autour de 50°C pour accélérer la solubilisation ;
C) refroidissement du mélange obtenu dans l'étape B) jusqu'à l'obtention d'une préparation parfaitement limpide et transparente ;
D) lorsque la préparation obtenue dans l'étape C) est parfaitement limpide, l'addition d'eau lentement et sous agitation afin de maintenir la limpidité de la préparation ;
E) l'addition à la préparation obtenue dans l'étape D) de l'acide 18-bêta-glycyrrhêtinique, sel dipotassique, et de l'allantoïne, agitation jusqu'à solubilisation complète ;
F) l'addition de l'acide acétique sous agitation à la préparation obtenue dans l'étape E) ;
G) l'ajustement du pH de la composition obtenue dans l'étape F) jusqu'à la valeur 4.

10. Méthode selon la revendication 9, **caractérisée en ce qu'**elle envisage éventuellement une étape supplémentaire H) à la suite de l'étape C) et avant l'étape D), dans laquelle le mélange des huiles essentielles est ajouté à la solution obtenue dans l'étape C) et solubilisé avec celle-ci.
